# EUROPÄISCHE PATENTSCHRIFT

(11) **EP 0 783 324 B1**
(45) Veröffentlichungstag und Bekanntmachung des Hinweises auf die Patenterteilung: **04.12.2002**
(21) Anmeldenummer: 95933299.0
(22) Anmeldetag: 26.09.1995
(51) Int. Cl.: A61K 47/48, A61P 37/04

(54) **KONJUGAT ZUR INDUKTION ZELLULÄRER IMMUNITÄT**
CELLULAR IMMUNITY-INDUCING CONJUGATE
CONJUGUE INDUISANT UNE IMMUNITE CELLULAIRE

(30) Priorität: 30.09.1994 DE 4435086
(43) Veröffentlichungstag der Anmeldung: 16.07.1997
(73) Patentinhaber: Deppert, Wolfgang Willi, 22359 Hamburg (DE)
(72) Erfinder: Deppert, Wolfgang Willi, 22359 Hamburg (DE)
(74) Vertreter: Schüssler, Andrea, Dr.
(86) Internationale Anmeldenummer: DE9501336
(87) Internationale Veröffentlichungsnummer: WO96010424

(56) Entgegenhaltungen:
- DATABASE CHEMABS CHEMICAL ABSTRACTS SERVICE, COLUMBUS, OHIO, US AN=123:167621, DEPPERT, WOLFGANG 'Conjugate containing a non-immunogenic carrier and a CD8+ T cell epitope for inducing cellular immunity especially against lymphocytic choriomeningitis virus' & DE,A,44 35 086 (DEPPERT, WOLFGANG)
- CANCER RESEARCH 54 (15). 1994. 4155-4161. ISSN: 0008-5472, MINEV B R ET AL 'Insertion signal sequence fused to minimal peptides elicits specific CD8+ T-cell responses and prolongs survival of thymoma -bearing mice.'
- PROC. NATL. ACAD. SCI. U. S. A. (1993), 90(8), 3530-4 CODEN: PNASA6;ISSN: 0027-8424, 1993 DONNELLY, JOHN J. ET AL 'Targeted delivery of peptide epitopes to class I major histocompatibility molecules by a modified Pseudomonas exotoxin'
- EUR. J. IMMUNOL. (1989), 19(9), 1657-67 CODEN: EJIMAF;ISSN: 0014-2980, 1989 SCHULZ, MANFRED ET AL 'Major histocompatibility complex-dependent T cell epitopes of lymphocytic choriomeningitis virus nucleoprotein and their protective capacity against viral disease' in der Anmeldung erwähnt
- J. IMMUNOL. (1994), 153(6), 2554-61 CODEN: JOIMA3;ISSN: 0022-1767, 1994 WEIDT, GUNNAR ET AL 'CD8+ T lymphocyte-mediated antiviral immunity in mice as a result of injection of recombinant viral proteins'
- DATABASE CHEMABS CHEMICAL ABSTRACTS SERVICE, COLUMBUS, OHIO, US VERJANS, GEORGES M. G. M. ET AL 'Intracellular processing and presentation of T cell epitopes, expressed by recombinant Escherichia coli and Salmonella typhimurium, to human T cells' & EUR. J. IMMUNOL. (1995), 25(2), 405-10 CODEN: EJIMAF;ISSN: 0014-2980, 1995
- DATABASE CHEMABS CHEMICAL ABSTRACTS SERVICE, COLUMBUS, OHIO, US FLYNN, J. N. ET AL 'Induction of feline immunodeficiency virus-specific cytotoxic T cells in vivo with carrier-free synthetic peptide' & J. VIROL. (1994), 68(9), 5835-44 CODEN: JOVIAM;ISSN: 0022-538X, 1994

## Beschreibung

Die vorliegende Erfindung betrifft ein Konjugat, das sich zur Induktion zellulärer Immunität eignet, sowie ein Verfahren zur Herstellung eines solchen Konjugats.

Eine der Hauptaufgaben des Immunsystems ist die Abwehr von Viren. Hierfür werden neben Antikörpern insbesondere CD8⁺ T Lymphozyten (nachstehend mit CD8⁺ T Zellen bezeichnet) als wichtig erachtet. Diesen wird eine wesentliche Rolle in der Erlangung zellulärer Immunität gegen Viren zugeschrieben.

Seit langer Zeit wird versucht, eine Immunität gegen Viren zu induzieren. Hierfür wird oftmals Vaccinia-Virus als Vektor verwendet. In sein Genom werden Gene von Viren integriert, gegen die eine zelluläre Immunität induziert werden soll. Das erhaltene rekombinante Vaccinia-Virus wird dann zur Expression der angesprochenen Gene sowie der Prozessierung ihrer Genprodukte in Versuchspersonen injiziert.

Hierbei hat sich jedoch häufig gezeigt, daß Vaccinia-Virus Neuropathien auslöste. Seine Verwendung zur Induktion zellulärer Immunität stellt daher ein nicht zu vertretendes Risiko dar.

Der vorliegenden Erfindung liegt somit die Aufgabe zugrunde, ein Mittel bereitzustellen, mit dem zelluläre Immunität gegen Viren induziert werden kann, ohne daß vorstehendes Risiko auftritt.

Erfindungsgemäß wird dies durch ein Konjugat erreicht, das einen im Körper nicht immunogen wirkenden Träger und ein CD8⁺-T Zell-Epitop umfaßt.

Der Ausdruck "im Körper nicht als immunogen wirkender Träger" umfaßt eine Verbindung jeglicher Art, die im Körper eines Tieres oder Menschen keine Immunreaktionen hervorruft. Eine solche Verbindung kann ein körpereigenes oder körperfremdes Protein bzw. ein Teil davon (Peptid) sein. Bevorzugt ist die Verbindung Serumalbumin, Fibrinogen oder Transferrin bzw. ein Peptid davon.

Der Ausdruck CD8⁺-T Zell-Epitop betrifft eine Verbindung jeglicher Art, die ein von CD8⁺-T Zellen erkennbares, d.h. MHC Klasse I-Molekül restringiertes, Epitop eines Antigens enthält. Solche Epitope sind bekannt. Beispielhaft wird auf nachstehende Epitope des LCM Virus (lymphocytic choriomeningitis virus) in Bezug auf BALB/c (H-2^{d})- bzw. B6 (H-2^{b})-Mäuse verwiesen:
L^{d}-restringiertes Nonapeptid [RPQASGVYM]
(Aminosäuren 118-126 von LCM Virus-Nukleoprotein)
(vgl. Schutz, M. et al., Eur. J. Immunol. 19 (1989), 1657
D^{b}-restringiertes Nonapeptid [AVYNFATCG]
(Aminosäuren 34-42 von LCM Virus-Glycoprotein C)
(vgl. Klavinskis, L.S. et al., Virology 178 (1990), 393; Gairin, J.E. et al., J. Virol. 66 (1992), 6755).

Darüberhinaus sind dem Fachmann Verfahren bekannt, erfindungsgemäß verwendbare Epitope zu identifizieren und bereitzustellen. Er wird sie vorzugsweise auf Antigenen von Viren, Bakterien, Einzellern oder Tumoren suchen. Auch sind für ihn Epitope von in Autoimmunreaktionen beteiligten Proteinen besonders interessant.

Ein erfindungsgemäßes Konjugat kann ein oder mehrere Epitope enthalten. Diese können gleich oder verschieden sein. Im letzteren Fall können z. B. Epitope von verschiedenen Antigenen eines oder mehrerer Viren bzw. von viralen und bakteriellen Antigenen gleichzeitig vorliegen.

Erfindungsgemäß können ein oder mehrere Epitope des Konjugats direkt oder über einen üblichen Linker an den Träger gebunden sein. Eine solche Bindung kann an einer beliebigen Stelle des Trägers sein. Bei mehreren Epitopen können diese zumindest teilweise getrennt voneinander oder als Einheit aneinander gereiht gebunden sein.

In bevorzugter Ausführungsform ist das erfindungsgemäße Konjugat ein Fusionsprotein. In einem solchen können ein oder mehrere Epitope an beliebiger Stelle des Träger-Proteins vorliegen. Bei mehreren Epitopen können diese zumindest teilweise in getrennter Form oder als Einheit aneinander gereiht sein. Besonders bevorzugt ist ein Fusionsprotein, in dem ein oder mehrere Epitope am N- und/oder C-Terminus des Träger-Proteins vorliegen.

Vorteilhaft kann es auch sein, wenn das erfindungsgemäße Konjugat ein oder mehrere Epitope aufweist, die in ihrer Sequenz im Vergleich zu jener im Antigen modifiziert sind. Solche Modifikationen können Additionen, Deletionen und/oder Substitutionen von ein oder mehreren Aminosäuren sein. Auch können einzelne Aminosäuren in derivatisierter Form vorliegen.

Desweiteren kann es günstig sein, wenn ein oder mehrere Epitope des erfindungsgemäßen Konjugats zusätzlich Aminosäuren der sie im Antigen flankierenden Sequenzen enthalten. Diese Aminosäuren können ebenfalls wie vorstehend modifiziert sein.

Darüberhinaus kann es vorteilhaft sein, wenn das erfindungsgemäße Konjugat geladene oder hydrophobe Gruppen aufweist. Dem Fachmann sind Verfahren bekannt, solche Gruppen in das Konjugat einzuführen.

Ferner ist darauf hinzuweisen, daß in dem erfindungsgemäßen Konjugat eine Homologie zwischen dem Träger und einem oder mehreren seiner Epitope bestehen kann. Bevorzugt ist allerdings ein Konjugat, indem der Träger eine Heterologie zu einem oder mehreren seiner Epitope aufweist.

Ein erfindungsgemäßes Konjugat kann in üblicher Weise hergestellt werden. Für ein Fusionsprotein erweist sich ein Verfahren mit folgenden Verfahrensschritten als günstig:
(a) Insertion einer für ein im Körper nicht immunogen wirkendes Träger-Protein codierenden DNA und einer für ein CD8⁺-T Zell-Epitop codierenden DNA im Leserahmen in einen Expressionsvektor,
(b) Transformation einer Zelle mit dem in (a) enthaltenen Expressionsvektor und Expression des Fusionsproteins, sowie
(c) Isolierung und Reinigung des in (c) erhaltenen Fusionsproteins.

Die Durchführung vorstehender Schritte ist dem Fachmann bekannt. Er kennt Vektoren und Zellen, um DNA-Sequenzen zu exprimieren. Beispielsweise wird er zur Expression in E. coli. den Vektor pH6EX3 verwenden (vgl. nachstehend). Für die Expression in Hefe wird er z.B. den Vektor pY100 und für die Expression in tierischen Zellen den Vektor pKCR einsetzen. Als geeignete Hefezellen wird er z.B. auf den Stamm Saccharomyces cerevisiae zurückgreifen und als eukaryotische Zellen z.B. CHO Zellen verwenden.

Desweiteren kennt der Fachmann auch Alternativen zu vorstehenden Verfahrensschritten, Beispielsweise ist ihm eine in Schritt (a) anzuwendende PCR Mutagenese geläufig. Mit diesem Verfahren kann die Insertion des CD8⁺-T Zell-Epitops in das Trägerprotein auf DNA-Ebene erfolgen (vgl. nachstehend).

Erfindungsgemäße Konjugate eignen sich zur Induktion zellulärer Immunität. Sie stellen einen neuen Typus einer Vakzine dar, die gegen die vielfältigsten Antigene prophylaktisch und therapeutisch eingesetzt werden kann. Typische Anwendungsmöglichkeiten der Vakzine liegen bei Viren, Bakterien und Einzellern. Besondere Bedeutung erfährt die Vakzine auch bei Tumor- und Autoimmunerkrankungen. Ihre ganz besondere Qualität zeigt sich darin, daß sie keinerlei Nebenwirkung verursacht.

Fig. zeigt erfindungsgemäße Fusionsproteine.

Die folgenden Beispiele erläutern die Erfindung.

### Beispiel 1: Herstellung erfindungsgemäßer Konjugate

Zur Herstellung erfindungsgemäßer Fusionsproteine wurde der für E. coli geeignete Expressionsvektor pH6EX3 verwendet (vgl. Berthold, H. M. et al., Prot. Expr. Purif. 3 (1992), 50). In diesen Vektor wurde die für NS-1₁₋₈₁ (Aminosäuren 1-81 des Nicht-Strukturproteins 1 von Influenza-Virus A), für TAg₂₋₂₇₀ (Aminosäuren 2-270 von SV 40 T-Ag) bzw. für Maus-Wildtyp p53 (Aminosäuren 2-387) codierende cDNA in üblicher Weise inseriert (vgl. Yamnikova, S.S. et al., Virology 197 (1993), 558; Brown, M.M. et al., J. Virol. 60 (1986) 290; Jenkins, J. R. et al., Nucl. Ac. Res. 12 (1984), 5609; Kolzau, T. und Deppert W., Int. J. Oncol. 3 (1993), 23). Nach Transformation von E. coli wurden die Expressionsprodukte N-0, S-0, T-0 und P-0 erhalten (vgl. Fig.).

In diese Expressionsprodukte wurden folgende LCM Virus CD8⁺ T Zell-Epitope eingebaut:
L^{d}-restringiertes Nonapeptid [RPQASGVYM]
(Aminosäuren 118-126 von LCM Virus-Nukleoprotein; vgl. vorstehend; nachstehend mit (E1) bezeichnet)
D^{b}-restringiertes Nonapeptid [AVYNFATCG]
(Aminosäuren 34-42 von LCM Virus-Glycoprotein C; vgl. vorstehend; nachstehend mit (E2) bezeichnet)
D^{b}-restringiertes Nonapeptid [VENPGGYCL]
(Aminosäuren 278-286 von LCM Virus-Glycoprotein C; nachstehend mit (E3) bezeichnet).

### 1. Orthotopischer Austausch von T-Zell-Epitopen

In T-0 wurde das eigene D^{b}-restringierte T Zell-Epitop (Aminosäuren 207-215) durch vorstehende Epitope (E1) bzw. (E2) ersetzt. Es wurden die Fusionsproteine T-1 und T-3 erhatten. Hierzu wurde zunächst die für T-0 codierende DNA einer üblichen PCR-Mutagenese unterzogen. Die erhaltene modifizierte DNA wurde dann kloniert und in E. coli exprimiert.

Desweiteren wurde in N-0 vorstehendes Epitop (E3) eingefügt. Es wurde das Fusionsprotein N-1 erhalten (vgl. Fig.). Hierzu wurde ebenfalls vorstehendes PCR-Mutagenese-Verfahren verwendet. Die Fusionsproteine T-1, T-3 und N-1 sind in der Fig. angegeben.

### 2. Heterotopische Einfügung von T-Zell-Epitopen

In T-0 wurden nahe des C-Terminus vorstehende T Zell-Epitope (E1) bzw. (E2) eingefügt. Es wurden die Fusionsproteine T-2 und T-4 erhalten. Hierzu wurde die für T-0 codierende DNA durch Insertion von Oligonukleotiden modifiziert. Die erhaltene DNA wurde kloniert und in E. coli exprimiert.

Ferner wurden in P-0 nahe des C-Terminus vorstehende T Zell-Epitope (E2) bzw. (E1) eingefügt. Es wurden die Fusionsproteine P-1 und P-2 erhalten.

Desweiteren wurde in S-0 nahe des C-Terminus vorstehendes T Zell-Epitop (E2) eingefügt. Es wurde das Fusionsprotein (S-1) erhalten.

Darüberhinaus wurde in T-0 nahe des N-Terminus vorstehendes T-Zell-Epitop (E2) eingefügt. Es wurde das Fusionsprotein (T-5) erhalten.

Ferner wurden in T-0 nahe des C-Terminus vorstehende T-Zell-Epitope (E2) und (E1) gemeinsam eingefügt. Es wurde das Fusionsprotein T-6 erhalten (vgl. Fig.). Vorstehende Fusionsproteine wurden in entsprechender Weise wie T-2 bzw. T-4 erhalten.

### Beispiel 2: Immunisierung von Mäusen mit erfindungsgemäßen Konjugaten

Die in Beispiel 1 erhaltenen Fusionsproteine wurden zusammen mit SDS in BALB/c- bzw. B6-Mäuse injiziert. Den Mäusen wurde dann LCM Virus verabreicht. Im einzelnen erhielten die Mäuse 14 Tage und 7 Tage vor Verabreichung des Virus jeweils 5µg Fusionsprotein. 5 Tage nach Virus-Verabreichung wurden infektiöse Titer aus Milzen der Mäuse bestimmt.

Es zeigte sich, daß in BALB/c-Mäusen T-0 und P-0 zu keinem Schutz vor LCM Virus führten. T-4 führte zu einem schwachen Schutz, während T-1, T-2 und T-6 sowie P-2 zu einem starken Schutz vor dem Virus führten (vgl. Tab. 1).

Desweiteren zeigte es sich, daß in B6-Mäusen T-0, P-0 und S-0 zu keinem Schutz vor LCM Virus führten. T-3, T-4, T-5 und T-6, sowie P-1 und S-1 führten jedoch zu einem großen Schutz vor dem Virus (vgl. Tab. 2).

Aus vorstehenden Daten geht hervor, daß ein Trägerprotein mit einem oder mehreren CD8⁺-T Zell-Epitopen zelluläre Immunität gegen Viren induzieren kann.

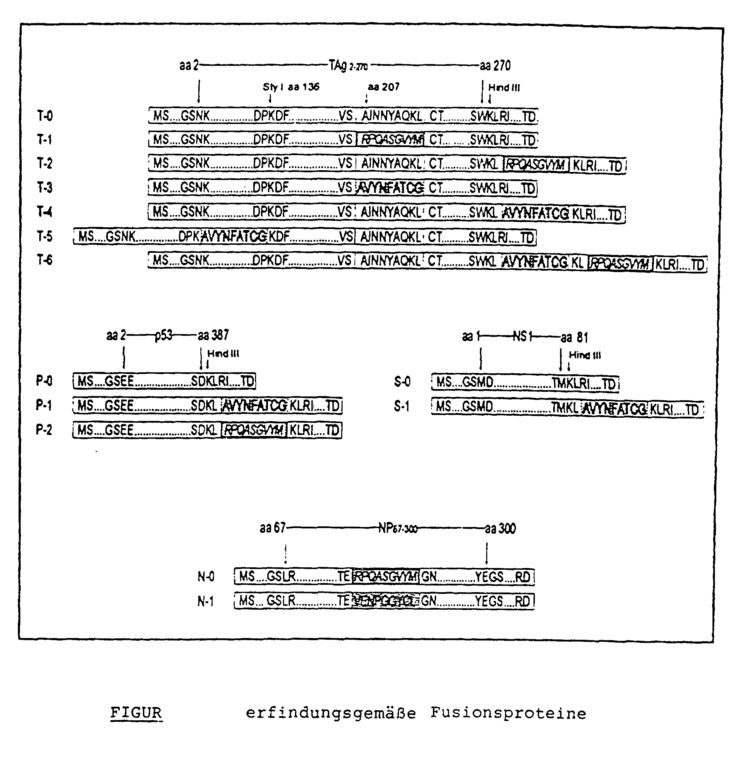

## Patentansprüche

1. Konjugat, umfassend einen im Körper nicht immunogen wirkenden Träger und ein CD8⁺-T-Zell-Epitop.

2. Konjugat nach Anspruch 1, **dadurch gekennzeichnet, daß** der Träger ein körpereigenes Protein oder ein Peptid davon ist.

3. Konjugat nach Anspruch 1, **dadurch gekennzeichnet, daß** der Träger ein körperfremdes Protein oder ein Peptid davon ist.

4. Konjugat nach einem der Ansprüche 1 bis 3, **dadurch gekennzeichnet, daß** mehrere gleiche oder verschiedene CD8⁺-T-Zell-Epitope vorliegen.

5. Konjugat nach einem der Ansprüche 1 bis 4, **dadurch gekennzeichnet, daß** das bzw. die CD8⁺-T-Zell-Epitope von einem Antigen eines Virus, Bakteriums, Einzellers und/oder Tumors stammen.

6. Konjugat nach einem der Ansprüche 1 bis 5, **dadurch gekennzeichnet, daß** es ein Fusionsprotein ist.

7. Verfahren zur Herstellung des Konjugats nach Anspruch 6, umfassend die folgenden Verfahrensschritte:
a) Insertion einer für ein im Körper nicht immunogen wirkendes Träger-Protein kodierenden DNA und einer für ein CD8⁺-T-Zell-Epitop kodierenden DNA im Leserahmen in einen Expressionsvektor,
b) Transformation einer Zelle mit dem in a) erhaltenen Expressionsvektor und Expression des Fusions-Proteins, sowie
c) Isolierung und Reinigung des in c) erhaltenen Fusions-Proteins.

8. Verwendung des Konjugats nach Anspruch 1 zur Herstellung einer pharmazeutischen Zusammensetzung zur Induktion zellulärer Immunität.

9. Verwendung nach Anspruch 8, wobei die Immunität gegen ein Virus, ein Bakterium, einen Einzeller und/oder einen Tumor gerichtet ist.

## Claims

1. A conjugate comprising a carrier which has no immunogenic effect within the body and a CD8⁺ T cell epitope.

2. The conjugate according to claim 1, **characterized in that** the carrier is a protein originating within the body or a peptide thereof.

3. The conjugate according to claim 1, **characterized in that** the carrier is a protein originating outside of the body or a peptide thereof.

4. The conjugate according to any of claims 1 to 3, **characterized in that** several equal or different CD8⁺ T cell epitopes are present.

5. The conjugate according to any of claims 1 to 4, **characterized in that** the CD8⁺ T cell epitope or epitopes are derived from an antigen of a virus, bacterium, protozoon and/or tumor.

6. The conjugate according to any of claims 1 to 5, **characterized in that** it is a fusion protein.

7. A method of producing the conjugate according to claim 6, comprising the steps of:
a) inserting a DNA coding for a carrier protein which has no immunogenic effect within the body and a DNA coding for a CD8⁺ T cell epitope in the reading frame in an expression vector,
b) transforming a cell with the expression vector obtained in a) and expressing the fusion protein, as well as
c) isolating and purifying the fusion protein obtained in c).

8. Use of the conjugate according to claim 1 for the production of a pharmaceutical composition for inducing cellular immunity.

9. Use according to claim 8, wherein the immunity is directed against a virus, a bacterium, a protozoon and/or a tumor.

## Revendications

1. Conjugué, comprenant un support n'exerçant pas d'action immunogène dans le corps et un épitope-cellule T-CD8⁺.

2. Conjugué selon la revendication 1, **caractérisé en ce que** le support est une protéine spécifique au corps ou un peptide de celle-ci.

3. Conjugué selon la revendication 1, **caractérisé en ce que** le support est une protéine étrangère au corps ou un peptide de celle-ci.

4. Conjugué selon la revendication 1 à 3, **caractérisé en ce que** plusieurs épitopes-cellule T-CD8⁺ identiques ou différents sont présents.

5. Conjugué salon l'une des revendications 1 à 4, **caractérisé en ce que** le ou les épitopes-cellule T- CD8⁺ proviennent d'un antigène d'un virus, d'une bactérie, d'un organisme monocellulaire et/ou d'une tumeur.

6. Conjugué selon l'une des revendications 1 à 5, **caractérisé en ce qu'**il s'agit d'une protéine de fusion.

7. Procédé pour la préparation du conjugué selon la revendication 6, comprenant les étapes de procédé suivantes :
a) insertion d'un ADN codant pour une protéine support n'exerçant pas d'action immunogène dans le corps et d'un ADN codant pour un épitope-cellule T-CD8⁺ dans le cadre de lecture dans le vecteur d'expression,
b) transformation d'une cellule avec le vecteur d'expression obtenu en a) et expression de la protéine de fusion, ainsi que
c) isolation et purification de la protéine de fusion obtenue en c).

8. Utilisation du conjugué selon la revendication 1 pour la préparation d'une composition pharmaceutique destinée à induire une immunité cellulaire.

9. Utilisation selon la revendication 8, selon laquelle l'immunité est dirigée contre un virus, une bactérie, un organisme monocellulaire et/ou une tumeur.
